**Europäisches Patentamt**

**European Patent Office**

**Office européen des brevets**

(11) Veröffentlichungsnummer: **0 038 531**
**A1**

(12) ## EUROPÄISCHE PATENTANMELDUNG

(21) Anmeldenummer: **81102917.2**

(22) Anmeldetag: **15.04.81**

(51) Int. Cl.³: **A 61 M 16/00**

(30) Priorität: **21.04.80 DE 3015287**

(43) Veröffentlichungstag der Anmeldung:
**28.10.81 Patentblatt 81/43**

(84) Benannte Vertragsstaaten:
**FR GB IT SE**

(71) Anmelder: **Siemens-Elema AB**
**Röntgenvägen 2**
**S-171 95 Solna 1(SE)**

(84) Benannte Vertragsstaaten:
**SE**

(71) Anmelder: **SIEMENS AKTIENGESELLSCHAFT Berlin**
**und München**
**Postfach 22 02 61**
**D-8000 München 22(DE)**

(84) Benannte Vertragsstaaten:
**FR GB IT**

(72) Erfinder: **Psardos, Georgios**
**A Salviusväg 122**
**S-146 00 Tullinge(SE)**

(54) **Lungenbeatmungsgerät.**

(57) Die Erfindung bezieht sich auf ein Lungenbeatmungsgerät mit einem Beatmungssystem, das ein Einatmungs- und ein Ausatmungsrohr sowie eine am Einatmungsrohr über einen Gasbalg angeschlossene Druckquelle für Beatmungsgas umfaßt, welcher Druckquelle ausgangsseitig ein Ventil zugeordnet ist, das in Abhängigkeit vom Volumen des Gasbalges den Gasfluß von der Druckquelle zum Gasbalg steuert. Ziel der Erfindung ist es ein Gerät zu schaffen, das hinsichtlich des Füllens und Leerens des Gasbalges klare Mischverhältnisse gestattet, insbesondere dann, wenn einem Patienten eine Mischung aus mehreren Gaskomponenten, z.B. Atemgas mit Narkosegas, zugeführt werden soll, deren Mischverhältnis nach Vorgabe, eines Sollwertes korrekt einstellbar sein soll. Die Betätigung des Ventiles sollte dabei gleichzeitig mit Mitteln geschehen die praktisch geräuschlos arbeiten. Dieses Ziel wird erfindungsgemäß dadurch erreicht, daß zwischen dem Gasbalg (4) und dem Ventil (10) ein Magnetsteuerelement (21, 22, 23) vorhanden ist, durch das in einer ersten Stellung des Gasbalges, in der dieser ein erstes, dem gefüllten Balgzustand zuzuordnendes Volumen aufweist, das Ventil im Sinne des Schließens steuerbar ist, und durch das in einer zweiten Stellung des Gasbalges, in der durch ein gegenüber dem ersten Volumen kleineres zweites Volumen des Balges ein Leerzustand signalisiert wird, das Ventil im Sinne einer Öffnung steuerbar ist.

./...

Croydon Printing Company Ltd.

FIG 1

SIEMENS AKTIENGESELLSCHAFT     Unser Zeichen
Berlin und München            VPA 80 P 5953 E

Lungenbeatmungsgerät

Die Erfindung bezieht sich auf ein Lungenbeatmungsgerät mit einem Beatmungssystem, das ein Einatmungs- und ein Ausatmungsrohr sowie eine am Einatmungsrohr über einen Gasbalg angeschlossene Druckquelle für Beatmungsgas umfaßt, welcher Druckquelle ausgangsseitig ein Ventil zugeordnet ist, das in Abhängigkeit vom Volumen des Gasbalges den Gasfluß von der Druckquelle zum Gasbalg steuert.

Ein Lungenbeatmungsgerät dieser Art ist durch den Siemens-Elema-Prospekt "Halothane Vaporizer 950, Enflurane Vaporizer 951", March 1979, bekannt, Bei diesem Gerät wird das Ventil speziell durch einen Hebel gesteuert, der an der unteren Seite des Balges schleift. Der Hebel bewegt den Ventilstößel so, daß mit abnehmendem Balgvolumen das Ventil zunehmend geöffnet und bei zunehmendem Balgvolumen entsprechend zunehmend geschlossen wird.

Nachteilig ist, daß sowohl in der Einatmungsphase, in der also ein angeschlossener Patient Atemgas aus dem Balg entnimmt, als auch in der Ausatmungsphase, wo also Gas von der Druckquelle in den Balg nachgeschoben wird, über das Ventil zum Gasbalg ein kontinuierlicher Gasfluß zustande kommt. Dieser kontinuierliche Gasfluß erlaubt keine korrekte Dosierung des Mischverhältnisses zwischen jeweiligem Restgas im Balg und dem Anteil, der neu hinzuzuführen ist. Es versteht sich von selbst, daß gerade dann, wenn das Atemgas erst aus mehreren Komponenten, z.B. Luft mit Sauerstoff oder Sauer-

Lst 5 Kli / 16.4.1980

stoff und Lachgas, zusammengemischt werden muß, die Einhaltung eines gewünschten Mischungswertes große Schwierigkeiten bereitet. Dieselben Schwierigkeiten treten auf, wenn zwischen Ventil und Gasbalg ein Narkosemischgerät (z.B. entsprechend DE-OS 27 06 192) eingeschaltet werden soll. Eine korrekte Dosierung des Narkosemischverhältnisses ist nur möglich, wenn die Narkoseflüssigkeit im Behälter mit einem vorgebbaren Druck des Atemgases belastet wird. Bei kontinuierlichem Gaszufluß läßt sich ein solcher Druck jedoch kaum einstellen. Als weiterer Nachteil ist anzusehen, daß der am Balg schleifende Hebel Schleifgeräusche erzeugt, die zu Belästigungen beim Patienten, insbesondere beim zu narkotisierenden Patienten, und Pflegepersonal führen.

Aufgabe der Erfindung ist es, ein Lungenbeatmungsgerät der eingangs genannten Art zu schaffen, das hinsichtlich des Füllens und Leerens des Gasbalges klare Mischverhältnisse gestattet, insbesondere dann, wenn dem Patienten eine Mischung aus mehreren Gaskomponenten, z.B. Atemgas mit Narkosezusatz, zugeführt werden soll, deren Mischverhältnis nach Vorgabe eines Sollwertes korrekt einstellbar sein soll. Die Betätigung des Ventiles sollte dabei gleichzeitig mit Mitteln geschehen, die praktisch geräuschlos arbeiten.

Die Aufgabe wird erfindungsgemäß dadurch gelöst, daß zwischen dem Gasbalg und dem Ventil ein Magnetsteuerelement vorhanden ist, durch das in einer ersten Stellung des Gasbalges, in der dieser ein erstes, dem gefüllten Balgzustand zuzuordnendes Volumen aufweist, das Ventil im Sinne des Schließens steuerbar ist, und durch das in einer zweiten Stellung des Gasbalges, in der durch ein gegenüber dem ersten Volumen kleineres zweites Volumen des Balges ein Leerzustand signalisiert wird, das Ventil im Sinne einer Öffnung steuerbar ist.

Die Erfindung gewährleistet klare Verhältnisse, wann der Balg gefüllt ist, so daß also kein weiteres Gas nachgeliefert werden muß, und wann er sich im Leerzustand befindet, auf den hin neues Gas nachgeliefert werden muß. Es ergibt sich somit ein Wechselstoßbetrieb zwischen Füllen des Balges und Leeren des Balges. Ein kontinuierlicher Fluß von Atemgas über das Ventil zum Gasbalg findet nicht mehr statt. Damit ergeben sich jedoch auch klare Verhältnisse, wenn das Atemgas beispielsweise aus mehreren Komponenten zusammengemischt werden soll. Das Gasgemisch läßt sich sehr viel leichter als bisher auf das gewünschte Mischverhältnis einstellen. Der Abgang von kontinuierlichem auf stoßweisen Betrieb erleichtert auch den Einsatz bei der Narkose. Am Lungenbeatmungsgerät kann dann in einfachster Weise im Gasweg zwischen Ventil und Gasbalg ein Mischgerät für Narkosegase (z.B. entsprechend DE-OS 27 06 192) angeschlossen werden. Ein solches Narkosemischgerät ist so gestaltet, daß beim Durchgang von Gas vor einer Düse zum Gasbalg ein Druck entsteht, aufgrund dessen Narkoseflüssigkeit aus dem Mischgerät in bestimmtem Mischverhältnis mit dem Atemgas zum Gasbalg gedrückt wird. Der erfindungsgemäß intermittierende Betrieb des Ventils am Ausgang der Druckquelle für das Atemgas schafft im Gegensatz zum kontinuierlichen Betrieb klare Druckverhältnisse. Damit ist eine feine Dosierung bei der Narkose gewährleistet und die Erfindung ist in optimaler Weise auch in Kombination mit Narkosemischgeräten anwendbar. Das gemäß der Erfindung vorgesehene Magnetsteuerelement arbeitet im Gegensatz zum schleifenden Hebel des Standes der Technik praktisch lautlos. Auch in diesem Sinne ist die Erfindung also sehr vorteilhaft.

Weitere Vorteile und Einzelheiten der Erfindung ergeben sich aus der nachfolgenden Beschreibung eines

- 4 -        VPA 80 P 5953 E

Ausführungsbeispieles anhand der Zeichnung und in Verbindung mit den Unteransprüchen.
Es zeigen:

Figur 1 ein Lungenbeatmungsgerät gemäß der Erfindung;

Figur 2 eine Ventilvariante des Lungenbeatmungsgerätes gemäß Figur 1.

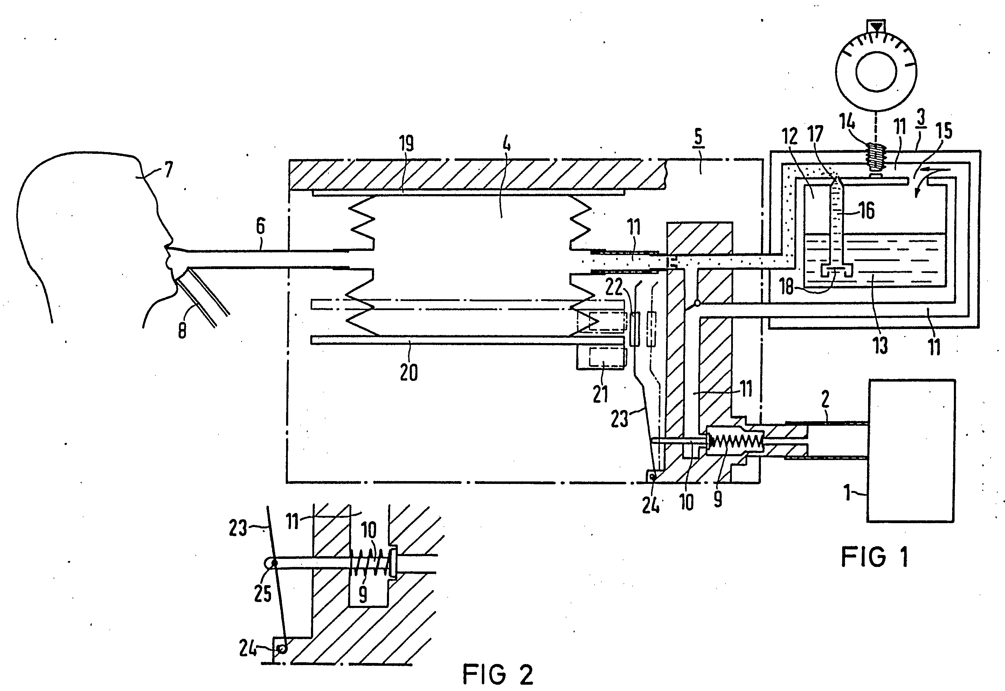

In der Figur 1 sind mit 1 ein Überdruckbehälter für Atemgas, z.B. $O_2$ oder $O_2/N_2O$-Mischung, und mit 2 der Gasausgang des Behälters bezeichnet. Das Gas der Druckquelle 1 soll über ein Narkosemischgerät 3 einem Gasbalg eines Beatmungsgerätes 5 zugeleitet werden. Am Balg 4 des Beatmungsgerätes 5 ist über ein Einatmungsrohr 6 ein Patient 7 angeschlossen. Die Ausatmung erfolgt über ein Ausatmungsrohr 8. Am Ausgang 2 des unter Überdruck stehenden Gasbehälters 1 liegt im Gaszuführweg zum Narkosemischgerät 3 ein mit einer Feder 9 belastetes Nadelventil 10. Die Gasverbindungsleitung ist mit 11 bezeichnet. Das im Gasweg eingeschaltete Narkosemischgerät 3 besitzt einen Behälter 12 für eine Narkoseflüssigkeit 13 mit leicht verdampfendem Narkosestoff. Der Behälter 12 ist im Gasweg vor einem von Hand einstellbaren Drosselventil 14 mit einer Öffnung 15 versehen, durch die Atemgas in den Behälter eintreten kann. Hierdurch wird Druck auf die Narkoseflüssigkeit 13 ausgeübt. Aufgrund dieses Druckes wird Narkoseflüssigkeit in bestimmter Dosierung durch eine Düse 16 zwecks Vergasung im Atemgas gedrückt, deren unteres Ende 18 in der Flüssigkeit liegt und deren oberes Düsenende 17 in der Gaszuführleitung 11 hinter dem Drosselventil 14 mündet. Der Gasbalg selbst ist im Beatmungsgerät 5 an dessen Decke mit der Stirnfläche 19 befestigt. Die untere bewegliche Stirnfläche ist mit 20 bezeichnet.

Gemäß der Erfindung sind nun sowohl dem Gasbalg 4 als auch dem Nadelventil 10 Magnetsteuermittel zugeordnet, die das Ventil schließen sollen, wenn der Balg 4 gefüllt ist und die das Ventil öffnen sollen, wenn der Balg geleert ist. Im vorliegenden Ausführungsbeispiel bestehen diese Magnetsteuermittel aus einem ersten Magneten 21, der an der unteren Stirnfläche 20 des Gasbalges 4 in der dargestellten Weise befestigt ist. In dessen Nähe befindet sich ein zweiter Magnet 22, der an einem Zungenhebel 23 befestigt ist, der um eine Achse 24 schwenkbar ist. Der Hebel 23 liegt am Stößel des Ventiles 10    an. Der Magnet 22 am Zungenhebel 23 ist so gepolt, daß er vom Magneten 21 abgestoßen wird, sobald er in den Bereich des Magnetfeldes gebracht wird. Außerhalb des Wirkungsbereiches des Magnetfeldes des Magneten 21 wird auf dem Magneten 22 hingegen keine Kraft ausgeübt.

Im Hinblick auf das Nadelventil 10 am Ausgang der Druckgasquelle 1 für das Atemgas ergibt sich damit folgende Wirkung:

Es wird angenommen, der Patient stehe unmittelbar vor einer Beatmung. Der Gasbalg 4 ist in diesem Augenblick gefüllt, d.h. die untere Stirnfläche 20 befindet sich in der dargestellten ausgezogenen Endstellung. In dieser Endstellung liegt jedoch der Magnet 22 am Zungenhebel 23 außerhalb des Wirkungsbereiches des Magneten 21 an der unteren Stirnfläche 20 des Balges 4. Der Magnet 22 wird also nicht abgestoßen; er befindet sich zusammen mit der Zunge 23 in der (fett) dargestellten Stellung. In dieser Stellung wird jedoch vom Zungenhebel 23 auf den Stößel des Nadelventils 10 keine Kraft ausgeübt; das Ventil 10 ist also geschlossen. Damit kann jedoch auch kein weiteres Gas aus dem Gasbehälter 1 in die Gasleitung 11 strömen.

Unmittelbar nachdem der Patient geatmet hat, befindet sich der Gasbalg 4 auf kleinerem Volumen (definiert als Leerzustand); die untere Stirnfläche 20 des Gasbalges 4 hat sich also nach oben bewegt und befindet sich jetzt in der punktgestrichelten Lage. In dieser Lage steht jedoch der Magnet 21 in unmittelbarer Nachbarschaft zum Magneten 22 des Zungenhebels 23. Damit kann der Magnet 21 mit seinem Magnetfeld voll auf den Magneten 22 wirken. Dieser wird also abgestoßen, wodurch auch der Zungenhebel 23 in die punktgestrichelte Stellung gebracht wird. In dieser Stellung drückt jedoch der Zungenhebel 23 auf den Stößel des Ventiles 10. Dieses ist jetzt gegen den Druck der Feder 9 geöffnet und es strömt Gas aus dem Überdruckbehälter 1 in die Gasleitung 11.

Das in der Gasleitung 11 strömende Gas erreicht nun zuerst das Narkosemischgerät 3. Je nach Einstellung des Drosselventils 14 wird über die Öffnung 15 auf die Narkoseflüssigkeit 13 ein mehr oder weniger starker Druck ausgeübt. Hierdurch wird mehr oder weniger Narkoseflüssigkeit über die Düsenöffnung 12 ausgespritzt. Die ausgespritzte Narkoseflüssigkeit verdampft sofort. Zum Balg 4 gelangt dann also ein Atemgas, das in vorgegebener Dosierung mit Narkosegas gemischt ist. Das in den Balg 4 eindringende Gemisch aus Atemgas und Narkosegas füllt den Balg 4 rasch, so daß er entsprechend rasch wieder seinen Füllzustand erreicht. Die untere Stirnfläche 20 des Gasbalges 4 befindet sich jetzt also wieder in der gezeichneten Stellung; der Magnet 21 hat keine Wirkung mehr auf den Magneten 22, so daß dieser samt Zungenhebel 23 in die gezeichnete Stellung zurückschnappt. Das Ventil 10 wird durch den Druck der Feder 9 rasch geschlossen. Dieser Vorgang wiederholt sich nun zyklisch im Takt der Atemzyklen des Patienten 7.

Damit ist also ein Beatmungsgerät geschaffen, mit dem einem Patienten in einfachster Weise und gut dosiert erwünschte Atemgasmischungen zugeleitet werden können. Das sich wechselweise im Stoßbetrieb öffnende und wieder schließende Ventil erlaubt, daß bei Anschluß eines Narkosemischgerätes im Behälter für die Narkoseflüssigkeit vorab ein genau dosierter Unterdruck eingestellt werden kann, der dazu führt, daß die ankommende Atemluft in genau dosierter Weise in den Behälter einströmen kann. Die in dosierter Weise einströmende Atemluft erzeugt dann einen genau dosierten Druck auf die Narkoseflüssigkeit, die dann entsprechend genau dosiert aus der Düsenöffnung 17 der Düse 16 ausgespritzt wird. Der Einsatz eines Ventilsteuermechanismus auf der Basis von Magneten gestattet also optimalen Einsatz des Beatmungsgerätes in Kombination mit einem Narkosemischgerät. Die spezielle Art der Magnetsteuerung führt aber zu einer geräuschlosen Arbeitsweise. Das sich öffnende und wieder schließende Ventil erzeugt praktisch keine Geräusche, so daß der Patient von dieser Seite her nicht mehr gestört wird. Der intermittierende Gasbetrieb erhöht zudem die Gesamtleistung des Lungenbeatmungsgerätes.

Die Figur 1 schildert lediglich ein einziges Ausführungsbeispiel. Selbstverständlich sind im Rahmen der Erfindung beliebige Modifikationen möglich. So können beispielsweise anstelle nur einer einzigen Atemgasquelle 1 mehrere Gasquellen vorgesehen sein. Dem Nadelventil 10 wird demnach also bereits ein Gasgemisch bestimmten Mischverhältnisses zugeführt. In Umkehrung des Abstoßprinzips der Magnetsteuermittel gemäß Figur 1 kann auch ein Prinzip angewendet werden, das mit Anziehung von Magneten arbeitet. Eine derartige Variante des Ventilsteuersystems der Figur 1 ist in der Figur 2 dargestellt. Hier ist das Nadelventil 10 am Zungenhebel 23 in einem Punkt 25 befestigt. Der Magnet 22 (nicht dargestellt) ist jetzt so gepolt, daß er vom Magneten 21 angezogen wird, sobald er in den Wirkungsbereich dessen Magnetfeldes gerät. Tritt also im Leerzustand des Gasbalges 4 der Balgmagnet 21 in Nachbarschaft zum Magneten 22 des Zungenhebels 23, so wird der Magnet 22 samt Hebel 23 angezogen. Der am Zungenhebel im Punkt 25 angelenkte Ventilstößel bewegt sich nach links und das Ventil öffnet gegen den Druck der Druckfeder 9. Im Füllzustand des Gasbalges 4 liegt der Magnet 22 des Zungenhebels 23 außerhalb des Wirkungsbereichs des Magnetfeldes des Balgmagneten 21. Der Magnet 22 wird also nicht angezogen; auf den Stößel des Ventils 10 wirkt also keine Kraft vom Zungenhebel 23 her, so daß das Ventil also durch den Druck der Druckfeder 9 in den geschlossenen Zustand gebracht wird.

2 Figuren
6 Patentansprüche

Patentansprüche

1. Lungenbeatmungsgerät mit einem Beatmungssystem, das ein Einatmungs- und ein Ausatmungsrohr sowie eine am Einatmungsrohr über einen Gasbalg angeschlossene Druckquelle für Beatmungsgas umfaßt, welcher Druckquelle ausgangsseitig ein Ventil zugeordnet ist, das in Abhängigkeit vom Volumen des Gasbalges den Gasfluß von der Druckquelle zum Gasbalg steuert, d a d u r c h g e k e n n z e i c h n e t , daß zwischen dem Gasbalg (4) und dem Ventil (10) ein Magnetsteuerelement (21, 22, 23) vorhanden ist, durch das in einer ersten Stellung des Gasbalges, in der dieser ein erstes, dem gefüllten Balgzustand zuzuordnendes Volumen aufweist, das Ventil im Sinne des Schließens steuerbar ist, und durch das in einer zweiten Stellung des Gasbalges, in der durch ein gegenüber dem ersten Volumen kleineres zweites Volumen des Balges ein Leerzustand signalisiert wird, das Ventil im Sinne einer Öffnung steuerbar ist.

2. Lungenbeatmungsgerät nach Anspruch 1, d a d u r c h g e k e n n z e i c h n e t , daß das Magnetsteuerelement aus wenigstens einem Magneten (21) und einem Element (22), das vom Magneten abgestoßen oder angezogen werden kann, besteht, wobei eines dieser beiden Bestandteile des Magnetsteuerelementes am Gasbalg (4) und das andere entsprechend an einem Betätigungsorgan (23) für das Ventil (10) angeordnet ist oder die beiden Teile Bestandteile des Balges oder des Betätigungsorganes sind und wobei die relative räumliche Anordnung beider Bestandteile (21, 22) zueinander so gewählt ist, daß in der Füllstellung des Gasbalges zwischen beiden Bestandteilen durch Abstoßen oder Anziehen ein erster Schaltzustand geschaffen ist, der über das Betätigungsorgan (23) zur Schließung des Ventils (10) führt und der in der Leerstellung des Gasbalges entsprechend umgekehrt durch Anziehen oder Abstoßen zu einer zweiten Kontaktstellung zwischen beiden Bestandteilen (21, 22) führt, die über das Betätigungsorgan (23) zum Öffnen des Ventiles führt.

3. Lungenbeatmungsgerät nach Anspruch 2, d a d u r c h g e k e n n z e i c h n e t , daß das Ventil (10) im Sinne des Öffnens oder Schließens durch ehen Schwenkhebel (23) gegen den Druck einer Druckfeder (9) betätigt wird, welcher Hebel (23) an seinem freien Ende mit dem einen Bestandteil (22) des Magnetsteuerelementes versehen ist, während der Balg (21) das andere Bestandteil (21) aufweist, und daß die relative räumliche Zuordnung beider Bestandteile (21, 22) vorzugsweise so ist, daß im Füllzustand des Gasbalges (4) beide Bestandteile (21, 22) nicht in direkter Nachbarschaft zueinander liegen, während im Leerzustand des Gasbalges (4) eine direkte räumliche Nachbarschaft zwischen beiden Bestandteilen (21, 22) gegeben ist.

4. Lungenbeatmungsgerät nach Anspruch 2 oder 3, d a d u r c h g e k e n n z e i c h n e t , daß das Element (22), das vom Magneten (21) angezogen oder abgestoßen werden soll, für den Fall des Anziehens als reines Metallstück oder als gleichsinnig gepolter Magnet oder im Falle des Abstoßens als gegensinnig gepolter Magnet ausgebildet ist.

5. Lungenbeatmungsgerät nach Anspruch 4, d a d u r c h g e k e n n z e i c h n e t , daß ein an der unteren Stirnfläche (20) des Gasbalges (4) angeordneter Magnet (21) durch die mit Füllen bzw. Leeren des Gasbalges (4) erzeugten Balgbewegungen in den Bereich des Gegenelementes (22) bewegbar bzw. aus dem Bereich wieder herausbewegbar ist.

- 11 -    VPA 80 P 5953 E

6. Lungenbeatmungsgerät nach einem der Ansprüche 1 bis 5, d a d u r c h g e k e n n z e i c h n e t , daß es zum Anschluß eines Narkosemischgerätes (3) zwischen Gasquelle (1) und Balg (4) ausgebildet ist.

FIG 1

FIG 2

Europäisches Patentamt

# EUROPÄISCHER RECHERCHENBERICHT

Nummer der Anmeldung

EP 81 10 2917.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - C - 1 095 469 (DRÄGERWERK) <br> * Fig. 1 * <br> -- | 1 |
| | DE - B2 - 2 537 307 (BIRD) <br> * Fig. 17 * <br> -- | 1 |
| | FR - A - 1 017 416 (J. CARRE) <br> * Fig. 1 * <br> -- | 1 |
| | FR - A - 1 240 954 (BRITISH OXYGEN) <br> * Fig. 2 * <br> -- | 1 |
| | FR - A - 1 240 955 (BRITISH OXYGEN) <br> * Fig. 1 * <br> -- | 1 |
| | FR - A - 1 553 667 (O.H. DRÄGER) <br> * Fig. 1 * <br> ---- | 1 |

**KLASSIFIKATION DER ANMELDUNG (Int. Cl.³)**

A 61 M 16/00

**RECHERCHIERTE SACHGEBIETE (Int. Cl.³)**

A 61 M 16/00
A 61 M 17/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 24-07-1981 | ZAPP |

EPA form 1503.1 06.78